# EUROPEAN PATENT SPECIFICATION

(11) **EP 4 110 229 B1**
(45) Date of publication and mention of the grant of the patent: **09.04.2025**
(21) Application number: 21713175.4
(22) Date of filing: 22.02.2021
(51) Int. Cl.: A61F 2/08

(54) **TENDON REPAIR DEVICE**
VORRICHTUNG ZUR REPARATUR VON SEHNEN
DISPOSITIF DE RÉPARATION DE TENDON

(30) Priority: 24.02.2020 US 202062980774 P
(43) Date of publication of application: 04.01.2023
(73) Proprietor: Smith & Nephew, Inc., Memphis, TN 38116 (US); Smith & Nephew Orthopaedics AG, 6300 Zug (CH); Smith & Nephew Asia Pacific Pte. Limited, Singapore 138565 (SG)
(72) Inventor: COTTON, Nicholas John, Mansfield, Massachusetts 02048 (US); TORRIE, Paul Alexander, Andover, Massachusetts 01810 (US)
(74) Representative: Appleyard Lees IP LLP
(86) International application number: PCT/US2021/019021
(87) International publication number: WO 2021/173483

(56) References cited:
- EP-A1- 0 358 324
- US-A- 5 441 508

## Description

### TECHNICAL FIELD

The present disclosure pertains generally, but not by way of limitation, to orthopedic implants and methods of treatment, said methods of treatment not forming part of the invention. More particularly, the present disclosure relates to a tendon repair implant, such as one that is engineered for arthroscopic placement over or in the area of a full or partial thickness tear of the rotator cuff tendons of the shoulder.

### BACKGROUND

With its complexity, range of motion and extensive use, a common soft tissue injury is damage to the rotator cuff or rotator cuff tendons. Damage to the rotator cuff is a potentially serious medical condition that may occur during hyperextension, from an acute traumatic tear or from overuse of the joint. Current procedures attempt to alleviate impingement or make room for movement of the tendon to prevent further damage and relieve discomfort but do not repair or strengthen the tendon. Use of the still damaged tendon can lead to further damage or injury. In some instances, biological tissue scaffolds are used to reinforce degenerated rotator cuff tendons. However, clinical outcomes are variable due to the chronicity and degree of degeneration of the tendon. An alternative technique may include superior capsule reconstruction (SCR), which utilizes biological tissue scaffolds to stabilize the joint by direct attachment of the glenoid to the humeral head. Accordingly, there is an ongoing need to design medical implants to treat injuries to the rotator cuff, rotator cuff tendons, or other soft tissue or tendon injuries throughout a body. An exemplary implant for a rotator cuff is disclosed in US 5,441,508.

### BRIEF SUMMARY

The invention provides a medical implant for treating a shoulder joint according to claim 1. Embodiments of the invention are defined in the dependent claims. For better understanding of the invention, the present disclosure further provides design, material, manufacturing method, and use alternatives for medical devices. An example medical implant for treating a shoulder joint includes a humeral head attachment region configured to be attached to a humeral head of the shoulder joint, a glenoid attachment region configured to be attached to a glenoid of the shoulder joint and a rotator cuff tendon attachment region configured to be attached to a rotator cuff tendon of the shoulder joint.

Alternatively or additionally to any of the embodiments above, wherein the humeral head attachment region is positioned between the glenoid attachment region and the rotator cuff tendon attachment region.

Alternatively or additionally to any of the embodiments above, wherein the glenoid attachment region is bifurcated into a first attachment arm and a second attachment arm, with a gap located between the first attachment arm and the second attachment arm.

Alternatively or additionally to any of the embodiments above, wherein the humeral head attachment region and the glenoid attachment region extend along a base of the implant with a bifurcation location therebetween.

Alternatively or additionally to any of the embodiments above, wherein the rotator cuff tendon attachment region extends from the bifurcation location at an acute angle to the base.

Alternatively or additionally to any of the embodiments above, wherein the glenoid attachment region is bifurcated into a first attachment arm and a second attachment arm, with a gap located between the first attachment arm and the second attachment arm.

Alternatively or additionally to any of the embodiments above, wherein the humeral head attachment region, the glenoid attachment region and the rotator cuff tendon attachment region together define a monolithic structure.

Alternatively or additionally to any of the embodiments above, wherein the humeral head attachment region includes a reinforcement member.

Alternatively or additionally to any of the embodiments above, further comprising one or more attachment supports extending from the reinforcement member, the one or more attachment supports configured to be anchored to the humeral head.

Alternatively or additionally to any of the embodiments above, wherein attachment of the humeral head attachment region to the humeral head and attachment of the glenoid attachment region to the glenoid is designed to pull the glenoid closer to the humeral head.

Another rotator cuff tendon repair implant includes a humeral head attachment member, a glenoid attachment member and a rotator cuff tendon attachment member each having a first end coupled to one another at a bifurcation region, and wherein each of the humeral head attachment member, the glenoid attachment member and the rotator cuff tendon attachment member have a second end which extends away from the bifurcation region.

Alternatively or additionally to any of the embodiments above, wherein the humeral head attachment member and the glenoid attachment member define a planar base having a first end, a second end and a first length extending therebetween, the bifurcation region located intermediate the first end of the base and the second end of the base, and wherein the rotator cuff tendon attachment member bifurcates from the base at the bifurcation region.

Alternatively or additionally to any of the embodiments above, wherein the glenoid attachment member is bifurcated into a first attachment arm and a second attachment arm.

Alternatively or additionally to any of the embodiments above, wherein the base and the rotator cuff tendon attachment member define a monolithic structure.

Alternatively or additionally to any of the embodiments above, wherein the base includes a first top surface adjacent to the glenoid attachment region and the rotator cuff tendon attachment member includes a bottom surface, and wherein the bottom surface is offset from the first top surface.

Alternatively or additionally to any of the embodiments above, wherein the bottom surface is offset from the first top surface at a first angle between 0 degrees and 30 degrees.

Alternatively or additionally to any of the embodiments above, wherein the base is configured to attach to a humeral head along the humeral head attachment region, the base is configured to attach to the glenoid along the glenoid attachment region and the rotator cuff tendon attachment member is configured to attach to a rotator cuff tendon along the rotator cuff tendon attachment region.

Alternatively or additionally to any of the embodiments above, wherein the rotator cuff attachment member is configured to attach to a bursal side of a rotator cuff.

Alternatively or additionally to any of the embodiments above, wherein the rotator cuff attachment member is configured to attach to the articular side of a rotator cuff.

A non-claimed method for delivering an implant to repair a shoulder includes advancing an implant to a target site, the implant including a humeral head attachment region, a glenoid attachment region and a rotator cuff tendon attachment region. The method further includes attaching the humeral head attachment region to a humeral head, attaching the glenoid attachment region to a glenoid and attaching the rotator cuff tendon attachment region to a rotator cuff tendon.

The above summary of some embodiments is not intended to describe each disclosed embodiment or every implementation of the present disclosure. The Figures, and Detailed Description, which follow, more particularly exemplify these embodiments.

### BRIEF DESCRIPTION OF THE DRAWINGS

The disclosure may be more completely understood in consideration of the following detailed description in connection with the accompanying drawings, in which:
FIG. 1 illustrates an example medical implant;
FIG. 2 is a side view of the medical implant shown in FIG.1;
FIG. 3 illustrates the medical implant of FIG. 1 implanted at a target site;
FIG. 4 illustrates the medical implant of FIG. 1 implanted at a target site;
FIG. 5 illustrates another example medical implant;
FIG. 6 illustrates another example medical implant;
FIG. 7 illustrates the medical implant of FIG. 6 implanted at a target site;
FIG. 8 illustrates the medical implant of FIG. 6 implanted at a target site;
FIG. 9 illustrates another example medical implant;
FIG. 10 illustrates the medical implant of FIG. 9 implanted at a target site;
FIG. 11 illustrates the medical implant of FIG. 9 implanted at a target site;
FIG. 12 illustrates the medical implant of FIG. 9 implanted at a target site;
FIG. 13 illustrates another example medical implant;
FIG. 14 illustrates the medical implant of FIG. 13 implanted to a target site;
FIG. 15 illustrates the medical implant of FIG. 13 implanted at a target site;
FIG. 16 illustrates the medical implant of FIG. 13 implanted at a target site;
FIG. 17 illustrates another example medical implant.

While the disclosure is amenable to various modifications and alternative forms, specifics thereof have been shown by way of example in the drawings and will be described in detail. It should be understood, however, that the intention is not to limit the disclosure to the particular embodiments described. The scope of the invention is defined by the appended claims.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary,

The following detailed description should be read with reference to the drawings in which similar elements in different drawings are numbered the same. The drawings, which are not necessarily to scale, depict illustrative embodiments and are not intended to limit the scope of the disclosure.

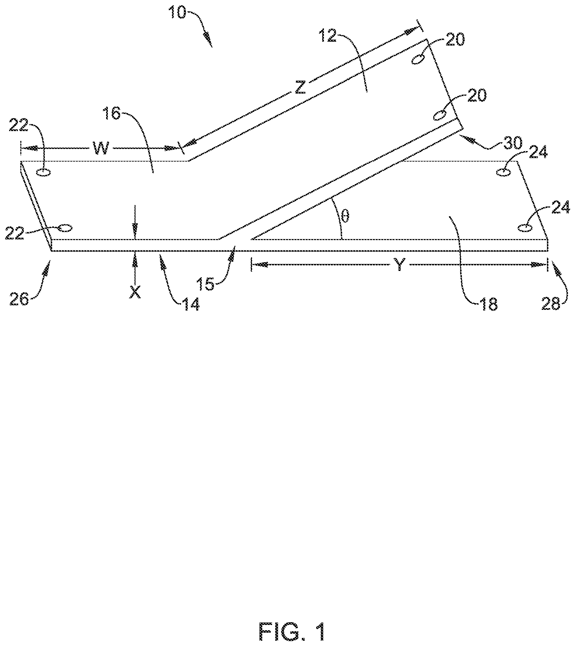

FIG. 1 illustrates an example tendon repair implant 10. As discussed above, the tendon repair implant 10 may be designed to repair damaged rotator cuff tendons of the shoulder. In some instances, the rotator cuff tendon repair implant 10 may be described as a scaffold structure having a base 14 and a rotator cuff attachment member 12 projecting away from the base 14 at a bifurcation location 15. As illustrated in FIG. 1, the base 14 may include a first end region 26 and a second end region 28 positioned opposite the first end region 26. The bifurcation location 15 may be positioned at an intermediate location between the first end region 26 and the second end region 28. Additionally, the rotator cuff attachment member 12 may include a third end region 30, which may be positioned at the free end of the rotator cuff attachment member 12 opposite the bifurcation location 15.

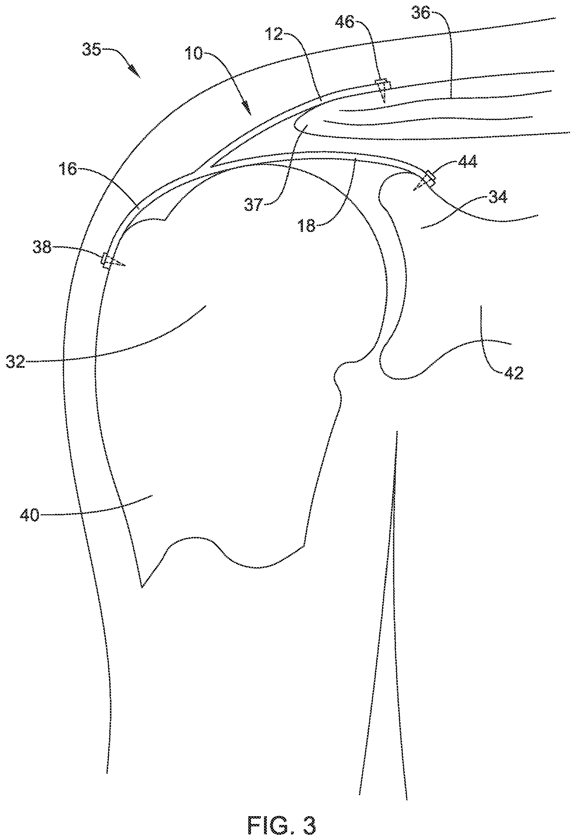

As illustrated in FIG. 1, the base 14 may include a humeral head attachment region 16 (located adjacent to or proximate the first end region 26) and a glenoid attachment region 18 (located adjacent to or proximate the first end region 28). It can be appreciated that the humeral head attachment region 16 may include that region of the tendon repair implant 10 which is designed to be attached to the humeral head of a patient. Similarly, it can be appreciated that the glenoid attachment region 18 may include that region of the tendon repair implant 10 which is designed to be attached to the glenoid of a patient. Further, the rotator cuff attachment member 12 of the tendon repair implant 10 may be designed to attach to a region of a rotator cuff tendon. The positioning and attachment of the tendon repair implant 10 will be shown in greater detail in FIGS. 3-4.

FIG. 1 further illustrates that the rotator cuff attachment member 12 may extend away from the base 14 at an angle θ. In some examples, the angle θ may be about 0 to about 30 degrees. Additionally, FIG. 1 illustrates that the glenoid attachment region 18 may have a length Y, which may be about 25 mm to about 50 mm. Further, FIG. 1 illustrates that the humeral head attachment region 16 may have a length W, which may be about 5 mm to about 30 mm. Further yet, FIG. 1 illustrates that the rotator cuff attachment member 12 may have a length Z, which may be about 10 mm to about 30 mm. Further yet, FIG. 1 illustrates that the base 14 (including the humeral head attachment region 16 and/or the glenoid attachment region 18) of the tendon repair device 10 may have a thickness X, which may be about 1 mm to about 5 mm.

Additionally, FIG. 1 illustrates that each of the glenoid attachment region 18, the rotator cuff attachment member 12 and the humeral head attachment region 16 may include one or more apertures designed to permit an anchor to extend therethrough and attach to a target site (e.g., glenoid, humeral head, tendons, etc.). For example, FIG. 1 illustrates that the glenoid attachment region 18 may include one or more apertures 24 located along the second end region 28 of the base 14, the humeral head attachment region 16 may include one or more apertures 22 located along the first end region 26 of the base 14 and the rotator cuff attachment member 12 may include one or more apertures 20 located along the third end region 30. While FIG. 1 illustrates that each of the glenoid attachment region 18, the humeral head attachment region 16 and the rotator cuff attachment member 12 may include two apertures, respectively, it is contemplated that, in some examples, each of the glenoid attachment region 18, the humeral head attachment region 16 and the rotator cuff attachment member 12 may include 1, 2, 3, 4, 5, 6, 7, 8 or more apertures positioned therein. In other instances, one or more of the glenoid attachment region 18, the humeral head attachment region 16 and the rotator cuff attachment member 12 may be otherwise configured for attachment to its respective anatomical structure with fasteners or anchors without the need or inclusion of apertures.

As described above, FIG. 1 illustrates that the rotator cuff attachment member 12 may be described as a flap, arm, branch, etc. which lies in a plane offset from a plane defined by the base 14. In other words, the configuration of the rotator cuff attachment member 12 may be defined as "non-planar" relative to the base 14. As will be described in greater detail below, the non-planar arrangement of the rotator cuff attachment member 12 and the base 14 may permit the rotator cuff attachment member 12 to attach to target treatment sites (e.g., bones, tendons, etc.) which are offset from the plane defining the base 14. However, it can be appreciated that, in some examples, the rotator cuff attachment member 12 may be formed from a flexible fabric material, and therefore, may be able to lay flat along the base 14 prior to implantation. It can further be appreciated that, after implantation, the rotator cuff attachment member 12 may assume a non-planar arrangement relative to the base 14.

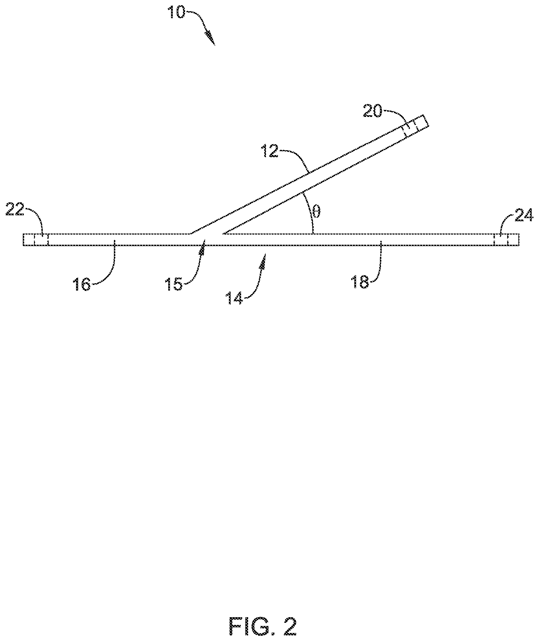

FIG. 2 illustrates a side view of the tendon repair device 10 described above with respect to FIG. 1. For example, FIG. 2 illustrates the humeral head attachment region 16 and the glenoid attachment region 18 of the base 14. FIG. 2 also illustrates the rotator cuff attachment member 12 attached to the base 14 at the bifurcation location 15 and extending away from the base 14 at an angle θ. FIG. 2 clearly illustrates that the plane in which the rotator cuff attachment member 12 lies is offset from and intersects the plane in which the base 14 lies (e.g., the rotator cuff attachment member 12 is non-parallel, and thus non-coplanar with respect to the base 14, as described above).

Additionally, as described above, FIG. 2 illustrates the apertures 24 extending through an end region of the glenoid attachment region 18 from an upper surface to a lower surface of the glenoid attachment region 18, the apertures 22 extending through an end region of the humeral head attachment region 16 from an upper surface to a lower surface of the humeral head attachment region, and the apertures 20 extending through an end region of the rotator cuff attachment member 12 from an upper surface to a lower surface of the rotator cuff attachment member 12.

FIG. 3 illustrates a cross-sectional view of a shoulder 35 including the example tendon implant device 10 described above implanted therein. The shoulder 35 further shows a head 32 of a humerus 40 mating with a glenoid 34 of the scapula 42. A rotator cuff tendon 36 is also shown. It can be appreciated from FIG. 3 that the end 37 of the rotator cuff tendon 36 has been retracted (e.g., torn away) from the humeral head 32. The muscles of the rotator cuff (along with others) control the movement of the humerus 40 relative to the scapula 42, and therefore, the separation of the rotator cuff tendon 36 from the humeral head 32 may create potential instability in the shoulder. As discussed above, to stabilize the shoulder, it may be desirable to not only reinforce the stability of the humeral head 32 relative to the glenoid 34, but also reinforce the stability of the rotator cuff tendon 36 to both the humeral head 32 and the glenoid 34 via the implant device 10.

FIG. 3 illustrates that the tendon repair device 10 may be positioned such that the humeral head attachment region 16 is adjacent to the humeral head 32, the glenoid attachment region 18 extends inferior to or underneath a portion of the rotator cuff 36 whereby it is positioned adjacent to the glenoid 34, and the rotator cuff attachment member 12 has been positioned such that it lies superior to or overtop (e.g., along the bursal side) of a portion of the rotator cuff tendon 36. Additionally, FIG. 3 illustrates that the humeral head attachment region 16 of the base 14 has been fixedly attached to the humeral head 32 via one or more anchors 38, the glenoid attachment region 18 of the base 14 has been fixedly attached to the glenoid 34 via one or more anchors 44 and the rotator cuff attachment member 12 has been attached to the rotator cuff 36 via one or more anchors 46.

It can be appreciated that while FIG. 3 illustrates the tendon repair device 10 attached to the humeral head 32, the glenoid 34 and/or the rotator cuff 36 utilizing anchors 38/44/46, it is contemplated that the device 10 may be attached to the humeral head 32, the glenoid 34 and/or the rotator cuff 36 using a variety of attachment mechanisms. For example, portions of the implant device 10 may be attached to the humeral head 32, the glenoid 34 and/or the rotator cuff 36 using staples, sutures, adhesive or the like.

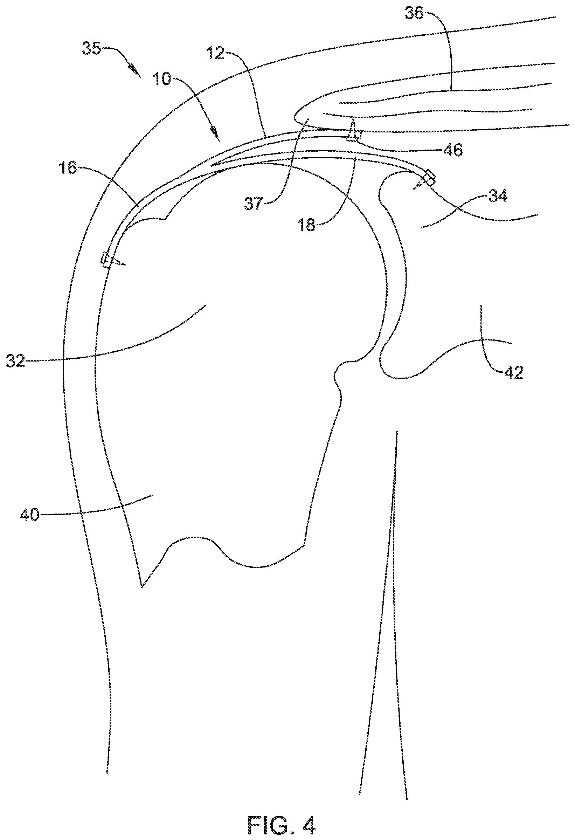

FIG. 4 illustrates an alternative attachment technique utilizing the tendon repair device 10 described above. FIG. 4 illustrates the tendon repair device 10 positioned substantially similar to that described above with respect to FIG. 3. For example, FIG. 4 illustrates that the humeral head attachment region 16 may be attached to the humeral head 32, the glenoid attachment region 18 may extend inferior to or underneath a portion of the rotator cuff 36 whereby it may be attached to the glenoid 34. However, in contrast to FIG. 3, FIG. 4 illustrates that, in some examples, the rotator cuff attachment member 12 may be attached to the articular side of the rotator cuff tendon 36. This attachment technique may be desirable if the surgeon wishes to work from inside the joint toward the exterior of the joint. FIG. 4 illustrates the anchoring mechanism (e.g., screws, staples, sutures, etc.) extending through a portion of the rotator cuff attachment member 12 and into the articular side of the rotator cuff tendon 36.

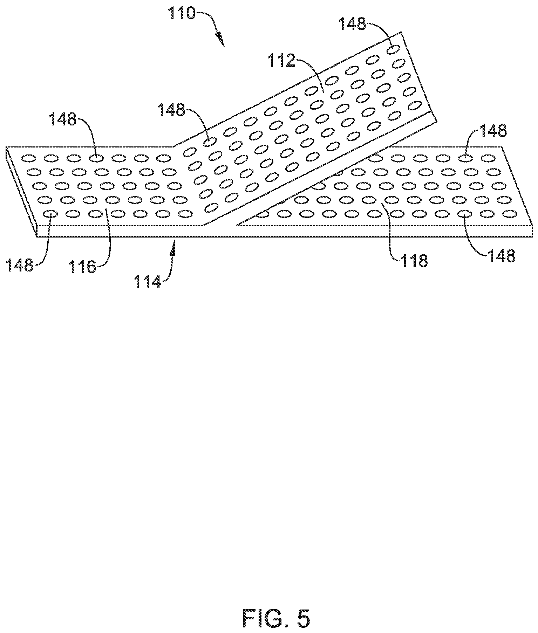

FIG. 5 illustrates another example tendon repair device 110. The tendon repair device 110 may be similar in form and function to the tendon repair device 10 described above. For example, the tendon repair device 110 may include a base 114 having a humeral head attachment region 116 designed to attach to a humeral head (as described above), a glenoid attachment region 118 designed to attach to a glenoid (as described above) and a rotator cuff attachment member 112 designed to attach to a rotator cuff tendon (as described above).

However, FIG. 5 illustrates that, in some examples, the implant 110 may include a multitude of apertures 148 distributed throughout the entire tendon repair device 110, or portions thereof. In some examples, the apertures 148 may be distributed uniformly throughout the implant 110. In other examples, the apertures 148 may be distributed unevenly throughout the implant 110. Designing the implant 110 to include a multitude of apertures distributed throughout the implant 110 may permit a clinician to attach the implant to a body structure (e.g., bone, tendon, etc.) through any one of the apertures 148, thereby lessoning the precision required for a clinician to attach the implant (and potentially decreasing the overall procedure time to implant the tendon repair device 110).

Further, it can be appreciated that any of the tendon repair devices disclosed herein may be constructed from a variety of materials. For example, the tendon repair devices disclosed herein may be constructed from a synthetic mesh. Other repair devices may be constructed from a synthetic mesh embedded in collagen. In yet other embodiments, the tendon repair devices may be constructed from a biodegradable material. Additionally, in some examples, the tendon repair devices disclosed herein may be constructed in a layered configuration, whereby a biological material is sequentially layered with a synthetic material.

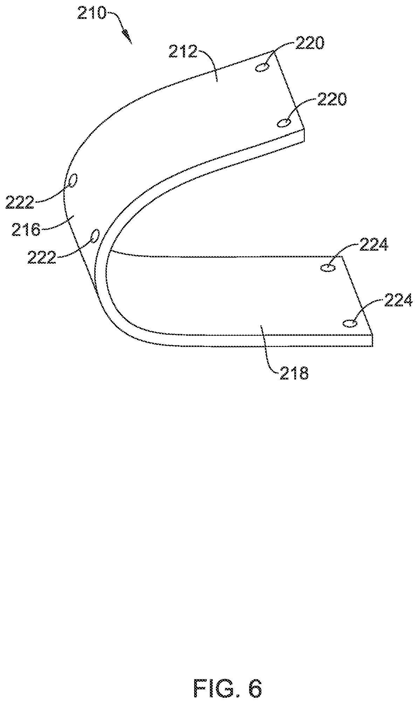

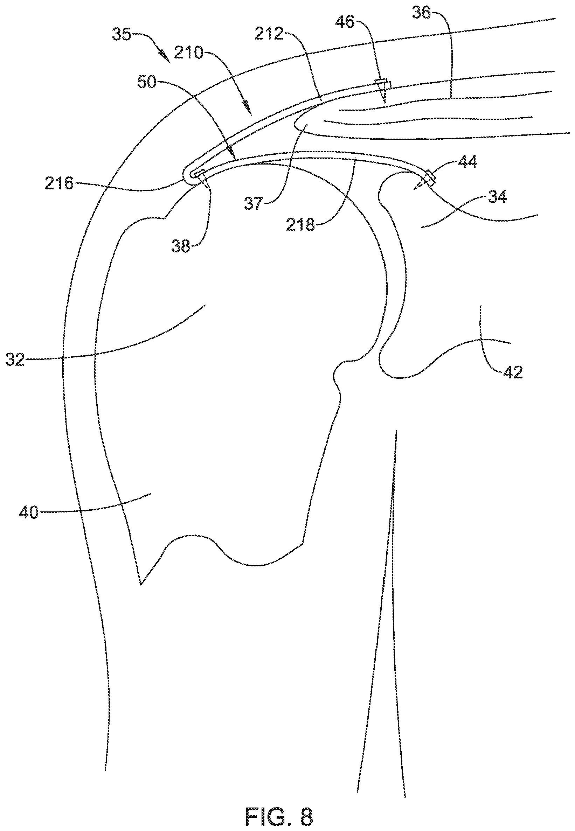

FIG. 6 illustrates another example tendon repair device 210. As discussed above, the tendon repair device 210 may be designed to repair damaged rotator cuff tendons of the shoulder. In some instances, the tendon repair device 210 may be a continuous member having a rotator cuff attachment region 212 and a glenoid attachment region 218. In some examples (such as that shown in FIG. 6), the tendon repair device 210 may "fold back" on itself to form a configuration in which it will be positioned in a patient's shoulder. The positioning and attachment of the tendon repair device 210 will be shown in greater detail in FIGS. 8-9.

Additionally, it can be appreciated that the tendon repair device 210 may further include a humeral head attachment region 216 positioned between the rotator cuff attachment region 212 and the glenoid attachment region 218. It can be appreciated that the humeral head attachment region 216 may include that portion of the tendon repair device 210 which is designed to be attached to the humeral head of a patient. Similarly, it can be appreciated that the glenoid attachment region 218 may include that portion of the tendon repair device 10 which is designed to be attached to the glenoid of a patient. Further, the rotator cuff attachment region 212 of the tendon repair device 210 may be designed to be attached to a portion of the rotator cuff tendons.

Additionally, FIG. 6 illustrates that each of the glenoid attachment region 218, the rotator cuff attachment region 212 and the humeral head attachment region 216 may include one or more apertures designed to permit an anchor to extend therethrough and attach to a target site (e.g., glenoid, humeral head, tendons, etc.). For example, FIG. 6 illustrates one or more apertures 224 positioned within the glenoid attachment region 218, one or more apertures 222 positioned within the humeral head attachment region 216 and one or more apertures 220 positioned within the rotator cuff attachment region 212. While FIG. 6 illustrates that each of the glenoid attachment region 218, the humeral head attachment region 216 and the rotator cuff attachment region 212 may include two apertures, respectively, it is contemplated that, in some examples, each of the glenoid attachment region 218, the humeral head attachment region 216 and the rotator cuff attachment region 212 may include 1, 2, 3, 4, 5, 6, 7, 8 or more apertures positioned therein. In other instances, one or more of the glenoid attachment region 218, the humeral head attachment region 216 and the rotator cuff attachment member 212 may be otherwise configured for attachment to its respective anatomical structure with fasteners or anchors without the need or inclusion of apertures.

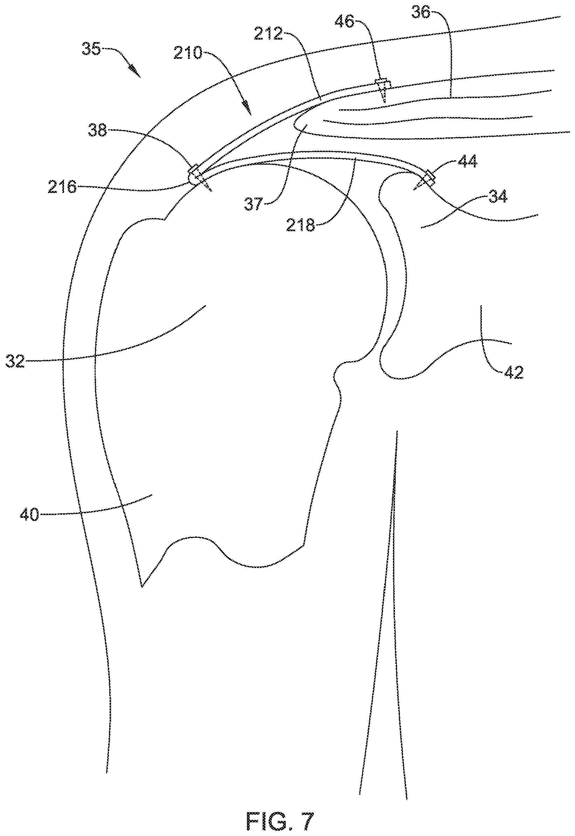

Like FIG. 3, FIG. 7 illustrates a cross-sectional view of a shoulder 35 including the example tendon implant device 210 described above. The shoulder 35 further shows a head 32 of a humerus 40 mating with a glenoid 34 of the scapula 42. A rotator cuff tendon 36 is also shown. It can be appreciated from FIG. 7 that the end 37 of the rotator cuff tendon 36 has been retracted (e.g., torn away) from the humeral head 32. The muscles of the rotator cuff (along with others) control the movement of humerus 40 relative to scapula 42, and therefore, the separation of the rotator cuff tendon 36 from the humeral head 32 may create potential instability in the shoulder. As discussed above, to stabilize the shoulder, it may be desirable to not only reinforce the stability of the humeral head 32 relative to the glenoid 34, but also reinforce the stability of the rotator cuff tendon 36 to both the humeral head 32 and the glenoid 34 via the implant device 210.

FIG. 7 illustrates that the tendon repair device 210 may be positioned such that the humeral head attachment region 216 is adjacent to the humeral head 32, the glenoid attachment region 218 extends inferior to or underneath a portion of the rotator cuff 236 whereby it is positioned adjacent to the glenoid 34, and the rotator cuff attachment region 212 has been positioned such that it lies superior to or overtop (e.g., along the bursal side) of a portion of the rotator cuff tendon 36. Additionally, FIG. 7 illustrates that the humeral head attachment region 216 has been fixedly attached to the humeral head 32 via one or more anchors 38, the glenoid attachment region 218 has been fixedly attached to the glenoid 34 via one or more anchors 44 and the rotator cuff attachment region 212 has been attached to the rotator cuff 36 via one or more anchors 46. For example, portions of the implant device 210 may be attached to the humeral head 32, the glenoid 34 and/or the rotator cuff 36 using staples, sutures, adhesive or the like.

FIG. 7 further illustrates that, according to the invention, the tendon repair device 210 is configured within the body such that tendon repair device 210 folds over on itself at the location where it is attached to the humeral head 32, with both the glenoid attachment region 218 and the rotator cuff attachment region 212 extending toward the sagittal plane of the patient from the humeral head attachment region 216. Further, it can be appreciated that the point at which the tendon repair device 210 folds over on itself may double the thickness of the device 210 at a location in which an anchor may attach the device 210 to the humeral head 32, in some instances. For example, FIG. 7 illustrates that the anchor 38 may extend through the folded over portion of the device 210 to anchor the humeral head attachment region 216 of the device 210 to the humeral head 32 with the anchor 38 passing through two thicknesses of the tendon repair device 210, if desired.

As discussed above, it can be appreciated that while FIG. 7 illustrates the tendon repair device 210 attached to the humeral head 32, the glenoid 34 and/or the rotator cuff 36 utilizing anchors 38/44/46, it is contemplated that the device 210 may be attached to the humeral head 32, the glenoid 34 and/or the rotator cuff 36 using a variety of attachment mechanisms. For example, portions of the implant device 10 may be attached to the humeral head 32, the glenoid 34 and/or the rotator cuff 36 using staples, sutures, or the like.

FIG. 8 illustrates an alternative attachment technique utilizing the tendon repair device 210 described above. FIG. 8 illustrates the tendon repair device 210 positioned substantially similar to that described above with respect to FIG. 7. For example, FIG. 8 illustrates that the glenoid attachment region 218 may extend inferior to or underneath a portion of the rotator cuff 36 whereby it may be attached to the glenoid 34 and the rotator cuff attachment region 212 may be positioned such that it lies superior to or overtop (e.g., along the bursal side) of a portion of the rotator cuff tendon 36. However, in contrast to FIG. 7, FIG. 8 illustrates that, in some examples, the anchor 38 may be positioned "inside" the folded over portion of the humeral head attachment region 216. In other words, FIG. 8 illustrates that the anchor 38 may be positioned along an inner surface 50 of the tendon repair device 210, whereby the anchor 38 extends through only a single thickness of the device 210 to attach to the humeral head 32.

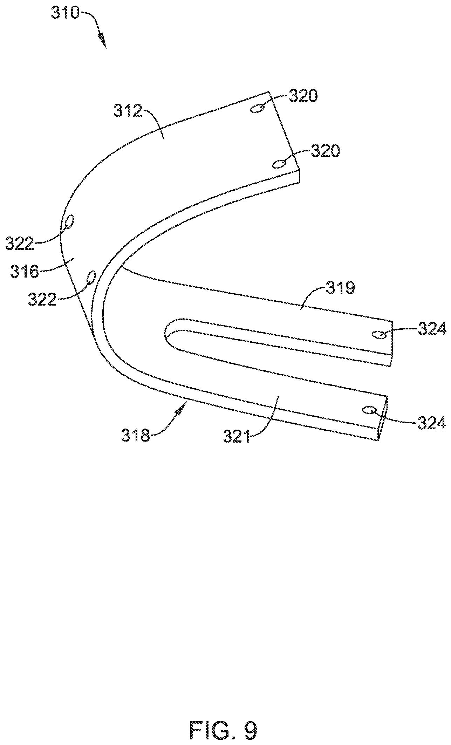

FIG. 9 illustrates another example tendon repair device 310. The tendon repair device 3 10 may be similar in form and function as the tendon repair device 210 described above. Like the device 210, the tendon repair device 310 may be a continuous member having a rotator cuff attachment region 312, a glenoid attachment region 318, and a humeral head attachment region 316 positioned between the rotator cuff attachment region 312 and the glenoid attachment region 318. However, as shown in FIG. 9, the glenoid attachment region 318 of the device 310 may be split or bifurcated into a first attachment arm 319 and a second attachment arm 321, with a gap or opening located therebetween.

Like other tendon repair devices described above, it can be appreciated that the humeral head attachment region 316 may include that portion of the tendon repair device 310 which is designed to attach to the humeral head of a patient. Similarly, it can be appreciated that the glenoid attachment region 318 may include that portion of the tendon repair device 310 which is designed to attach to the glenoid of a patient. Further, the rotator cuff attachment region 312 of the tendon repair device 310 may be designed to attach to a portion of the rotator cuff tendons.

Additionally, FIG. 9 illustrates that the first attachment arm 319 and the second attachment arm 321 of the glenoid attachment region 318, the rotator cuff attachment region 312 and the humeral head attachment region 316 may include one or more apertures designed to permit an anchor to extend therethrough and attach to a target site (e.g., glenoid, humeral head, tendons, etc.). For example, FIG. 9 illustrates one or more apertures 324 positioned within the first attachment arm 319 and the second attachment arm 321 of the glenoid attachment region 318, one or more apertures 322 positioned within the humeral head attachment region 316 and one or more apertures 320 positioned within the rotator cuff attachment region 312. In other instances, one or more of the glenoid attachment region 318, the humeral head attachment region 316 and the rotator cuff attachment region 312 may be otherwise configured for attachment to its respective anatomical structure with fasteners or anchors without the need or inclusion of apertures.

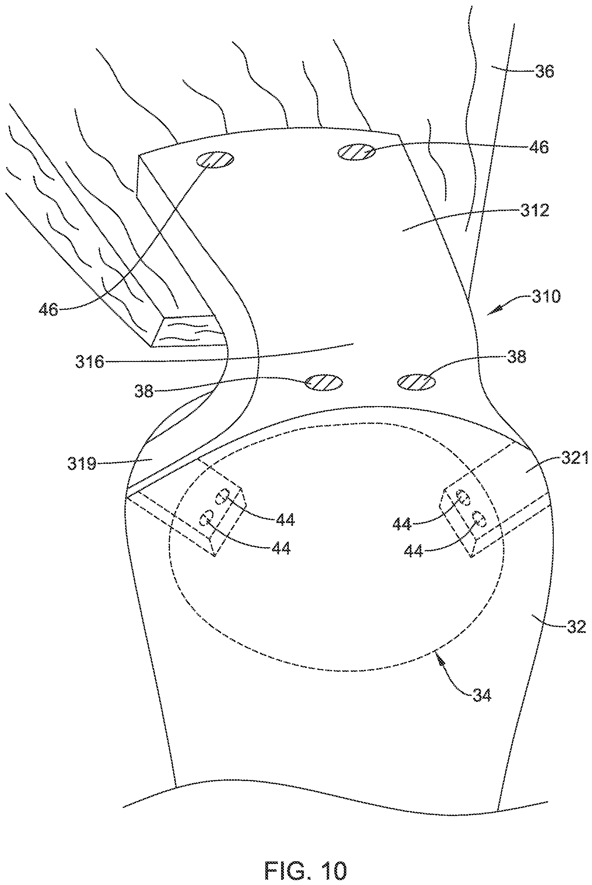

FIG. 10 illustrates a perspective view of the example tendon repair device 310 implanted within a shoulder. It can be appreciated that the placement and anchoring techniques utilized for the rotator cuff attachment region 312 and the humeral head attachment region 318 of the repair device 310 may be similar in form and function to the tendon repair device 210 described with respect to FIGS. 6-8. For example, FIG. 10 shows the rotator cuff attachment region 312 attached to the rotator cuff 36 via one or more anchors 46. Additionally, FIG. 10 shows the humeral head attachment region 316 anchored to the humeral head 32 via one or more anchors 38.

However, FIG. 10 further illustrates that attachment of the first attachment arm 319 and the second attachment arm 321 of the glenoid attachment region 318 may "wrap around" or extend around the sides of the humeral head 32 at a position inferior to a superiormost extent of the humeral head 32 and attach themselves along anterior and posterior sides of the glenoid 34 (depicted as the dashed line 34) via one or more anchors 44. The anchor points 44 can be more inferior than anchor points described in previous embodiments and may provide a downward restraint vector between the humeral head 32 and the glenoid 34. Further, if the glenoid 34 is interpreted to represent the face of a clock when viewed laterally, it can be appreciated that the first arm 319 and the second arm 321 may be attached at locations represented by approximately 10:00 and 2:00, for example.

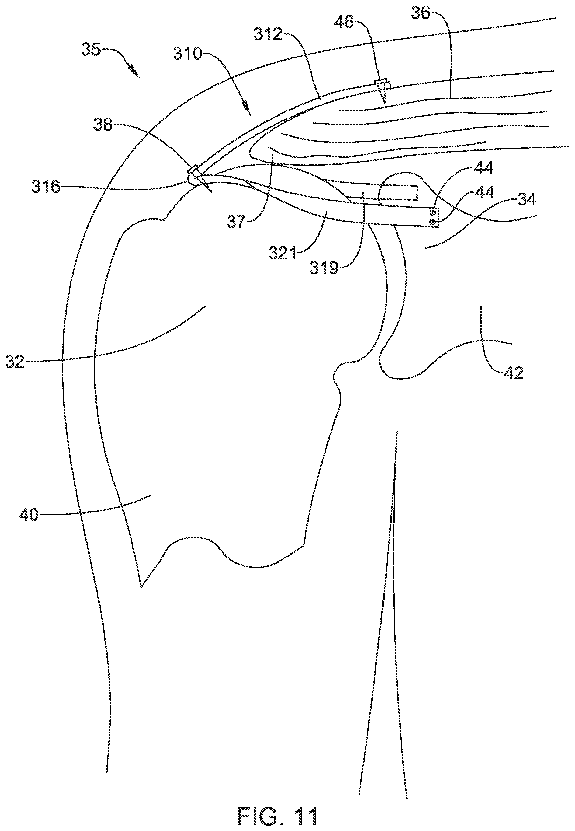

FIG. 11 illustrates a cross-sectional view of a shoulder 35 including the example tendon implant device 310 described above. The shoulder 35 further shows a head 32 of a humerus 40 mating with a glenoid 34 of the scapula 42. A rotator cuff tendon 36 is also shown. FIG. 11 further illustrates that the tendon repair device 310 may be positioned such that the humeral head attachment region 316 is attached to the humeral head 32 via one or more anchors 38 and the rotator cuff attachment member 312 has been positioned such that it is attached to the rotator cuff tendon 36, such as the bursal side the rotator cuff tendon 36, via one or more anchors 46. Further, FIG. 11 illustrates the first attachment arm 319 and the second attachment arm 321 of the glenoid attachment region 318 "wrapping around" or extending around the humeral head 32 at a position inferior to the humeral head anchors 38, whereby each of the first attachment arm 319 and the second attachment arm 321 are attached to the glenoid via anchors 44.

It can be appreciated from FIG. 11 that the design and corresponding attachment technique utilized with the tendon repair device 310 may be permit the distal end 37 of the rotator cuff tendon 36 to be pulled directly onto the humeral head 32. For example, the bifurcated design of the glenoid attachment region 318 (e.g., the ability of the first attachment arm 319 to separate from the second attachment arm 321) may permit the distal end 37 of the rotator cuff tendon 36 to be positioned directly against the humeral head 32 in the gap of opening between the first and second attachment arms 319, 321. Allowing the rotator cuff tendon 36 to be positioned directly against the humeral head 32 may facilitate the direct re-attachment of the rotator cuff tendon 36 to the humeral head 32.

Furthermore, as shown in FIGS. 10 and 11, it can be seen that the attachment point of the humeral head region 316 to the humeral head 32 (i.e., via anchors 38) is located superior to the attachment points of the first and second arms 319, 321 of the glenoid attachment region 318 (i.e., via anchors 44), thus generating a force vector that pulls the humeral head 32 in an inferior direction toward the sagittal plane of the patient.

Like the tendon repair device 210 described with respect to FIG. 7, FIG. 11 further illustrates that, in some examples, the tendon repair device 310 may be configured within the body such that tendon repair device 310 folds over on itself at the location where it is attached to the humeral head 32. Further, it can be appreciated that the point at which the tendon repair device 310 folds over on itself may double the thickness of the device 310 at a location in which an anchor may attach the device 310 to the humeral head 32. For example, FIG. 11 illustrates that the anchor 38 may extend through the folded over portion of the device 310, and thus through two thicknesses of the device 310, to anchor the humeral head attachment region 316 of the device 310 to the humeral head 32.

As discussed above, it can be appreciated that while FIG. 11 illustrates the tendon repair device 310 attached to the humeral head 32, the glenoid 34 and/or the rotator cuff 36 utilizing anchors 38/44/46, it is contemplated that the device 310 may be attached to the humeral head 32, the glenoid 34 and/or the rotator cuff 36 using a variety of attachment mechanisms. For example, portions of the implant device 310 may be attached to the humeral head 32, the glenoid 34 and/or the rotator cuff 36 using staples, sutures, or the like.

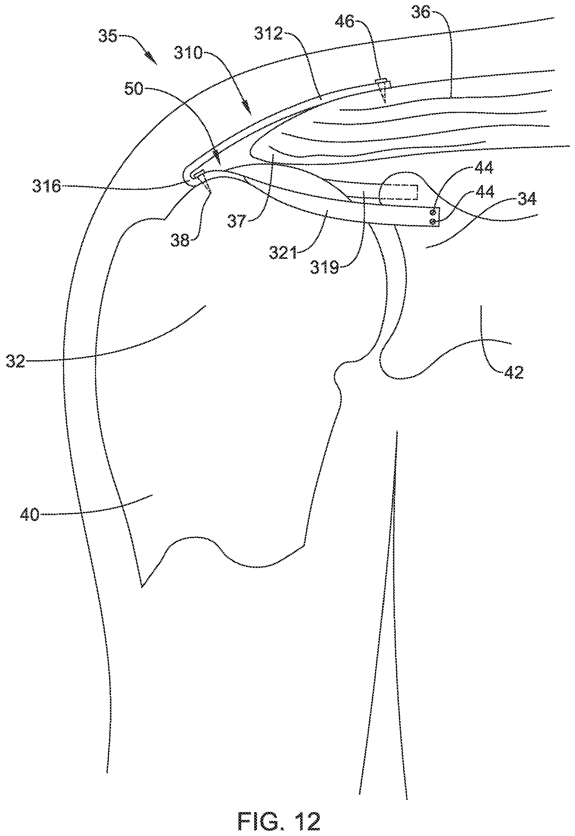

Like the tendon repair device 210 described with respect to FIG. 9, FIG. 12 illustrates an alternative attachment technique utilizing the tendon repair device 310 described above. FIG. 12 illustrates the tendon repair device 310 positioned substantially similar to that described above with respect to FIG. 11. For example, FIG. 12 illustrates that the first attachment arm 319 and the second attachment arm 321 of the glenoid attachment region 318 "wrapping around" the humeral head 32 and attaching to the glenoid 34. FIG. 12 also illustrates that the rotator cuff attachment member 312 may be positioned such that it lies overtop (e.g., along the bursal side) of a portion of the rotator cuff tendon 36. However, in contrast to FIG. 11, FIG. 12 illustrates that, in some examples, the anchor 38 may be positioned "inside" the folded over portion of the humeral head attachment portion 316. In other words, FIG. 12 illustrates that the anchor 38 may be positioned along an inner surface 50 of the tendon repair device 310, whereby the anchor 38 extends through only a single thickness of the device 310 to attach to the humeral head 32.

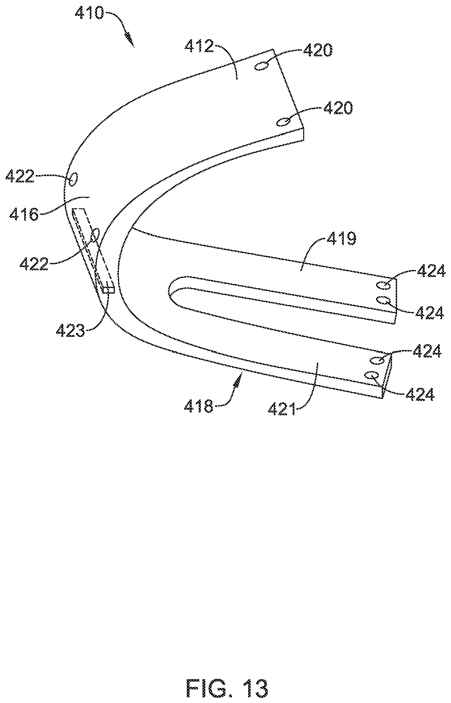

FIG. 13 illustrates another example tendon repair device 410. The tendon repair device 410 may be similar in form and function as the tendon repair device 310 described above. Like the device 310, the tendon repair device 410 may be a continuous member having a rotator cuff attachment member 412, a humeral head attachment region 416 and a glenoid attachment region 418 split or bifurcated into a first attachment arm 419 and a second attachment arm 421, with a gap or opening located therebetween.

Additionally, FIG. 13 illustrates that the first attachment arm 419 and the second attachment arm 421 of the glenoid attachment region 418, the rotator cuff attachment member 412 and the humeral head attachment region 416 may include one or more apertures designed to permit an anchor to extend therethrough and attach to a target site (e.g., glenoid, humeral head, tendons, etc.). For example, FIG. 13 illustrates one or more apertures 424 positioned within the first attachment arm 419 and the second attachment arm 421 of the glenoid attachment region 418, one or more apertures 422 positioned within the humeral head attachment region 416 and one or more apertures 420 positioned within the rotator cuff attachment region 412. In other instances, one or more of the glenoid attachment region 418, the humeral head attachment region 416 and the rotator cuff attachment region 412 may be otherwise configured for attachment to its respective anatomical structure with fasteners or anchors without the need or inclusion of apertures.

Additionally, FIG. 13 illustrates that the tendon repair device 410 may further include a reinforcement member 423 positioned within or along the humeral head attachment region 416. In some examples, the reinforcement member 423 may be embedded within the region of the tendon repair device defined by the humeral head attachment region 416. As shown in FIG. 13, the reinforcement member 423 may extend across the width of the tendon repair device 410. Further, in some examples, the reinforcement member 423 may be shaped similar to a rectangular beam. However, it is contemplated that the reinforcement member 423 may include different shapes. For example, it is contemplated that the reinforcement member 423 may be circular, square, ovular, etc. Additionally, it is contemplated that the repair device 410 may include multiple, smaller reinforcement members. For example, it can be appreciated that the reinforcement member 423 may be divided into multiple, smaller reinforcement members.

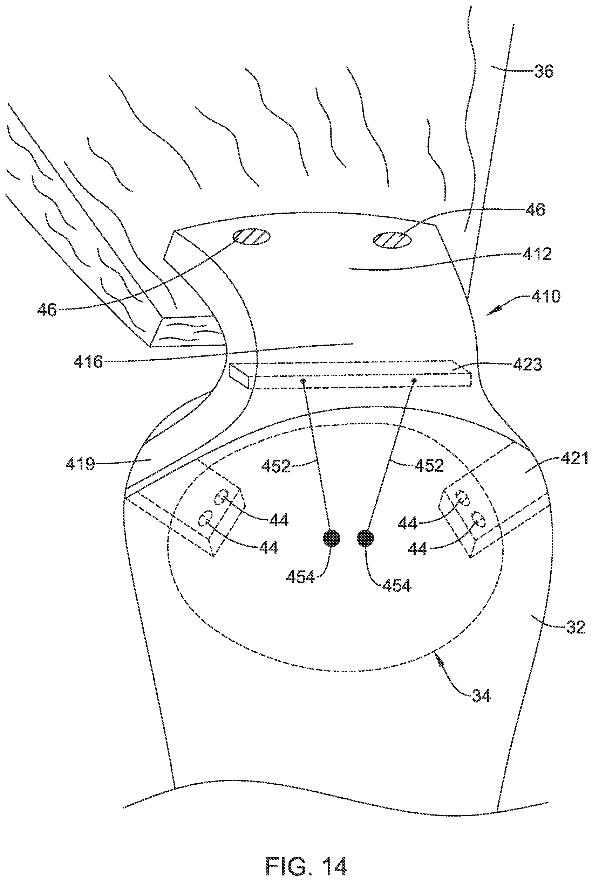

FIG. 14 illustrates a perspective view of the example tendon repair device 410 implanted within a shoulder. It can be appreciated that the placement and anchoring techniques utilized for the rotator cuff attachment region 412 and the humeral head attachment region 416 of the repair device 410 may be similar in form and function to the tendon repair device 310 described with respect to FIGS. 10-12. For example, FIG. 14 shows the rotator cuff attachment region 412 attached to the rotator cuff 36 via one or more anchors 46. FIG. 14 further illustrates that attachment of the first attachment arm 419 and the second attachment arm 421 of the glenoid attachment region 418 may "wrap around" or extend around the sides of the humeral head 32 at a position inferior to a superiormost extent of the humeral head 32 and attach themselves along anterior and posterior sides of the glenoid 34 (depicted as the dashed line 34) via one or more anchors 44.

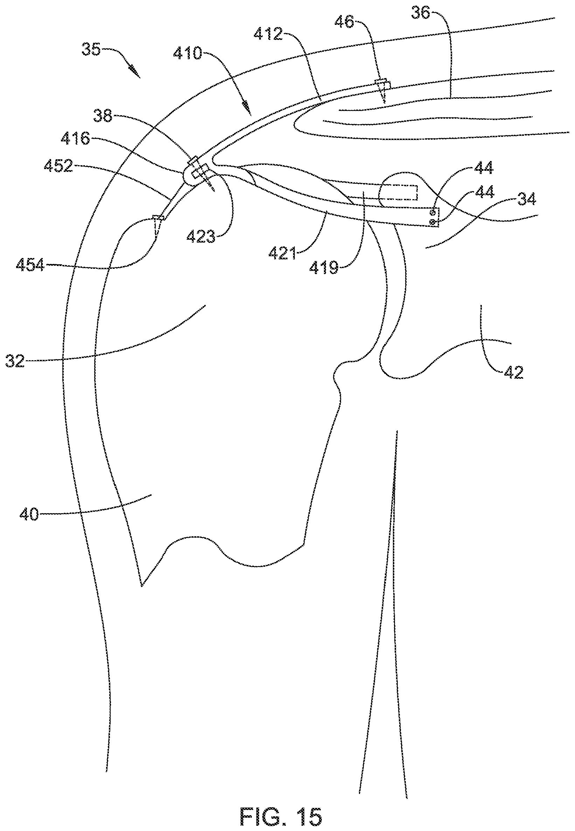

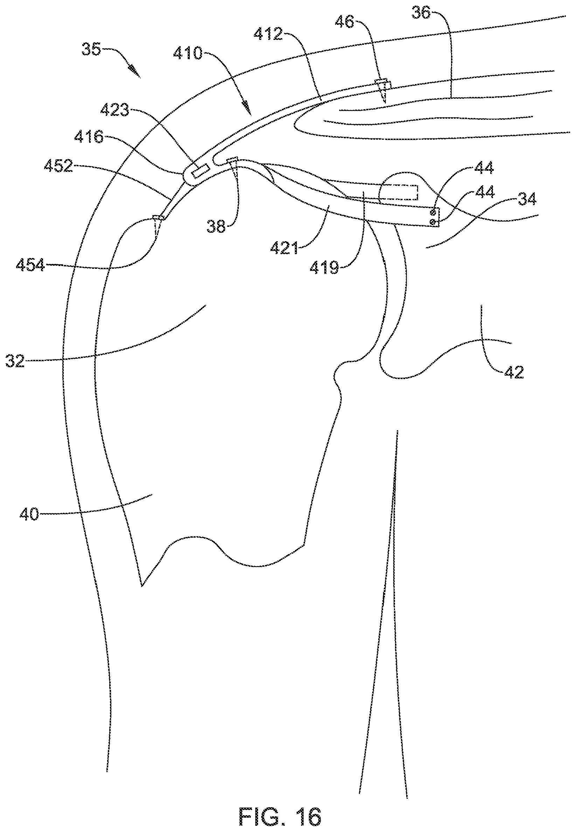

Additionally, FIG. 14 illustrates that the tendon repair device 410 may further include one or more attachment supports 452 which may be designed to extend from the reinforcement member 423 (positioned along the humeral head attachment region 416) to the humeral head 32 (FIG. 15 and FIG. 16 will illustrate the positioning of the attachment supports 452 in greater detail). It can be appreciated from FIG. 14 that each of the attachment supports 452 may have a first end which is attached to the reinforcement member 423 and a second end (opposite the first end) which is attached to the humeral head 32 via anchors 454.

Like the tendon repair device 310 described with respect to FIG. 11, FIG. 15 illustrates a cross-sectional view of a shoulder 35 including the example tendon implant device 410 described above. The shoulder 35 further shows a head 32 of a humerus 40 mating with a glenoid 34 of the scapula 42. A rotator cuff tendon 36 is also shown. FIG. 15 illustrates that the rotator cuff attachment region 412 has been positioned such that it is attached to the rotator cuff tendon 36, such as the bursal side the rotator cuff tendon 36, via one or more anchors 46. Further, FIG. 15 illustrates the first attachment arm 419 and the second attachment arm 421 of the glenoid attachment region 418 "wrapping around" or extending around the humeral head 32 at a position inferior to a superiormost extent of the humeral head 32, whereby each of the first attachment arm 319 and the second attachment arm 421 are attached to the glenoid via anchors 44.

Additionally, FIG. 15 further illustrates that the tendon repair device 410 may be positioned such that the humeral head attachment region 416 is attached to the humeral head 32 via one or more anchors 38. Further, FIG. 15 illustrates that the anchor 38 may extend through the reinforcement member 423 positioned within the humeral head attachment region (as described above). Alternatively, the anchor 38 may lay adjacent (e.g., medial) to the reinforcement member 423. Additionally, FIG. 15 illustrates the attachment supports 452 extending between the reinforcement member 423 and the humeral head 32. It can be appreciated from FIG. 15 that the attachment supports 452 may be attached to the humeral head 32 via the anchors 454. It can be appreciated that the attachment supports 452 may distribute the load transmitted into the humeral head 32 over a larger surface area.

FIG. 16 illustrates the same cross-sectional view of a shoulder 35 including the example tendon implant device 410 described above with respect to FIG. 15. For example, the shoulder 35 shows a head 32 of a humerus 40 mating with a glenoid 34 of the scapula 42. A rotator cuff tendon 36 is also shown. FIG. 16 illustrates that the rotator cuff attachment member 412 has been positioned such that it is attached to the bursal side the rotator cuff tendon 36 via one or more anchors 46. Further, FIG. 16 illustrates the first attachment arm 419 and the second attachment arm 421 of the glenoid attachment region 418 "wrapping around" or extending around the humeral head 32 at a position inferior to a superiormost extent of the humeral head 32, whereby each of the first attachment arm 319 and the second attachment arm 421 are attached to the glenoid via anchors 44. The anchors 44 being located inferior to the anchors 38 to generate a force vector pulling the humeral head 32 toward the glenoid 34 in an inferior direction toward the sagittal plane of the patient.

Additionally, FIG. 16 further illustrates that the tendon repair device 410 may be positioned such that the humeral head attachment region 416 is attached to the humeral head 32 via one or more anchors 38. However, in contrast to FIG. 15 above, FIG. 16 illustrates that the anchor 38 may positioned along the inner surface 50 of the tendon repair device 410. Like FIG. 15, however, FIG. 16 illustrates the attachment supports 452 extending between the reinforcement member 423 and the humeral head 32. It can be appreciated from FIG. 16 that the attachment supports 452 may be attached to the humeral head 32 via the anchors 454.

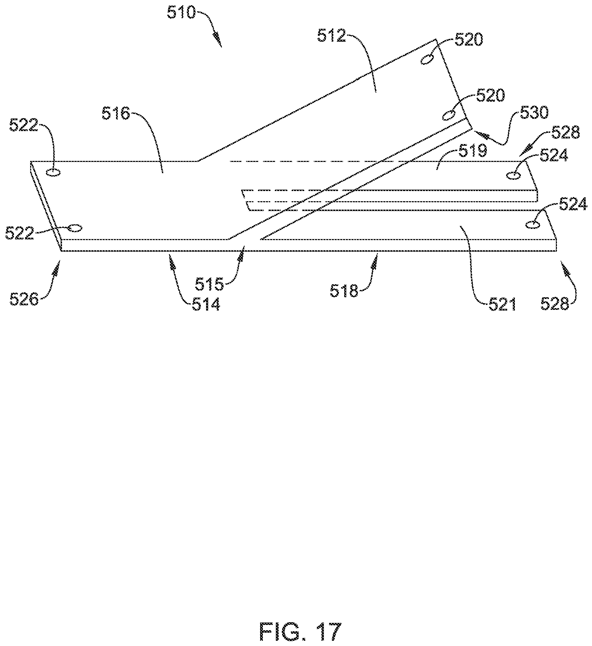

FIG. 17 illustrates another example tendon repair implant 510. The example tendon repair implant 510 may be similar in form and function to the tendon repair implant 10 discussed with respect to FIG. 1 and may also include features which are similar to the tendon repair implants discussed with respect to FIGS. 9-16.

As discussed above, the tendon repair implant 510 may be designed to repair damaged rotator cuff tendons of the shoulder. In some instances, the rotator cuff tendon repair implant 510 may be described as a scaffold structure having a base 514 and a rotator cuff attachment member 512 projecting away from the base 514 at a bifurcation location 515. As illustrated in FIG. 17, the base 514 may include a first end region 526 and a second end region 528 positioned opposite the first end region 526. The bifurcation location 515 may be positioned at an intermediate location between the first end region 526 and the second end region 528. Additionally, the rotator cuff attachment member 512 may include a third end region 530, which may be positioned at the free end of the rotator cuff attachment member 512 opposite the bifurcation location 515.

As illustrated in FIG. 17, the base 514 may include a humeral head attachment region 516 (located adjacent to or proximate the first end region 526) and a glenoid attachment region 518 (located adjacent to or proximate the first end region 528). It can be appreciated that the humeral head attachment region 516 may include that region of the tendon repair implant 510 which is designed to be attached to the humeral head of a patient. Similarly, it can be appreciated that the glenoid attachment region 518 may include that region of the tendon repair implant 510 which is designed to be attached to the glenoid of a patient. Further, the rotator cuff attachment member 512 of the tendon repair implant 510 may be designed to attach to a region of a rotator cuff tendon.

Further, as shown in FIG. 17, the glenoid attachment region 518 of the device 510 may be split or bifurcated into a first attachment arm 519 and a second attachment arm 521, with a gap or opening located therebetween. As discussed above with respect to FIGS. 9-11, the first attachment arm 519 and the second attachment arm 52 1 of the glenoid attachment region 518 may "wrap around" or extend around the sides of the humeral head at a position inferior to a superiormost extent of the humeral head and attach themselves along anterior and posterior sides of the glenoid.

Additionally, the first attachment arm 519 and the second attachment arm 521 of the glenoid attachment region 518, the rotator cuff attachment region 512 and the humeral head attachment region 516 may include one or more apertures designed to permit an anchor to extend therethrough and attach to a target site (e.g., glenoid, humeral head, tendons, etc.). For example, FIG. 17 illustrates one or more apertures 524 positioned within the first attachment arm 519 and the second attachment arm 521 of the glenoid attachment region 518, one or more apertures 522 positioned within the humeral head attachment region 516 and one or more apertures 520 positioned within the rotator cuff attachment region 512. In other instances, one or more of the glenoid attachment region 518, the humeral head attachment region 516 and the rotator cuff attachment region 512 may be otherwise configured for attachment to its respective anatomical structure with fasteners or anchors without the need or inclusion of apertures.

While FIG. 17 illustrates that the humeral head attachment region 516 and the rotator cuff attachment member 512 may include two apertures, and that each attachment arm 519/521 of the glenoid attachment region 518 may include one aperture, respectively, it is contemplated that, in some examples, each of the glenoid attachment region 518, the humeral head attachment region 516 and the rotator cuff attachment member 512 may include 1, 2, 3, 4, 5, 6, 7, 8 or more apertures positioned therein. In other instances, one or more of the glenoid attachment region 518, the humeral head attachment region 516 and the rotator cuff attachment member 512 may be otherwise configured for attachment to its respective anatomical structure with fasteners or anchors without the need or inclusion of apertures.

Like the tendon implant 10 described above this respect to FIG. 1, FIG. 17 illustrates that the rotator cuff attachment member 512 may be described as a flap, arm, branch, etc. which lies in a plane offset from a plane defined by the base 514. In other words, the configuration of the rotator cuff attachment member 512 may be defined as "non-planar" relative to the base 514. As has been previously described, the non-planar arrangement of the rotator cuff attachment member 512 and the base 514 may permit the rotator cuff attachment member 512 to attach to target treatment sites (e.g., bones, tendons, etc.) which are offset from the plane defining the base 514.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The scope of the invetnion is defined by the appended claims.

## Claims

1. A medical implant (210, 310, 410) for treating a shoulder joint, comprising:
a humeral head attachment region (216, 316, 416) configured to be attached to a humeral head of the shoulder joint;
a glenoid attachment region (218, 318, 418) configured to be attached to a glenoid of the shoulder joint; and
a rotator cuff tendon attachment region (212, 312, 412) configured to be attached to a rotator cuff tendon of the shoulder joint,
wherein the medical implant (210, 310, 410) is a continuous member, wherein the humeral head attachment region (216, 316, 416) is positioned between the glenoid attachment region (218, 318, 418) and the rotator cuff tendon attachment region (212, 312, 412);
wherein the medical implant is configured to fold over on itself in within the body at a position along the humeral head attachment region (216, 316, 416) with both the glenoid attachment region (218, 318, 418) and the rotator cuff tendon attachment region (212, 312, 412) extending toward the sagittal plane from the humeral head attachment region (216, 316, 416).

2. The medical implant (310, 410) of claim 1, wherein the glenoid attachment region (318, 418) is bifurcated into a first attachment arm (319, 419) and a second attachment arm (321, 421), with a gap located between the first attachment arm (318, 418) and the second attachment arm (319, 419).

3. The medical implant (210, 310, 410) of any one of claims 1-2, wherein the humeral head attachment region (216, 316, 416), the glenoid attachment region (218, 318, 418) and the rotator cuff tendon attachment region (212, 312, 412) together define a monolithic structure.

4. The implant (410) of any one of claims 1-3, wherein the humeral head attachment region (416) includes a reinforcement member (423).

5. The implant (410) of claim 4, further comprising one or more attachment supports (452) extending from the reinforcement member (423), the one or more attachment supports (452) configured to be anchored to the humeral head.

6. The medical implant (210, 310, 410) of any preceding claim, wherein each of the glenoid attachment region (218, 318, 418), the rotator cuff attachment region (212, 312, 412), and the humeral head attachment region (216, 316, 416) includes one or more apertures (220, 222, 224, 320, 322, 324, 420, 422, 424) configured to permit an anchor to extend therethrough and to attach to a target site.

## Patentansprüche

1. Medizinisches Implantat (210, 310, 410) zur Behandlung eines Schultergelenks, umfassend:
einen Humeruskopfanbringungsbereich (216, 316, 416), der zum Anbringen an einem Humeruskopf des Schultergelenks ausgestaltet ist,
einen Glenoidanbringungsbereich (218, 318, 418), der zum Anbringen an einem Glenoid des Schultergelenks ausgestaltet ist, und
einen Rotatorenmanschettensehnenanbringungsbereich (212, 312, 412), der zum Anbringen an einer Rotatorenmanschettensehne des Schultergelenks ausgestaltet ist,
wobei das medizinische Implantat (210, 310, 410) ein durchgehendes Glied ist, wobei der Humeruskopfanbringungsbereich (216, 316, 416) zwischen dem Glenoidanbringungsbereich (218, 318, 418) und dem Rotatorenmanschettensehnenanbringungsbereich (212, 312, 412) positioniert ist,
wobei das medizinische Implantat dazu ausgestaltet ist, sich in dem Körper an einer Position entlang des Humeruskopfanbringungsbereichs (216, 316, 416) auf sich selbst umzufalten, wobei sich sowohl der Glenoidanbringungsbereich (218, 318, 418) als auch der Rotatorenmanschettensehnenanbringungsbereich (212, 312, 412) von dem Humeruskopfanbringungsbereich (216, 316, 416) zu der Sagittalebene hin erstrecken.

2. Medizinisches Implantat (310, 410) nach Anspruch 1, wobei der Glenoidanbringungsbereich (318, 418) in einen ersten Anbringungsarm (319, 419) und einen zweiten Anbringungsarm (321, 421) gegabelt ist, wobei sich ein Spalt zwischen dem ersten Anbringungsarm (318, 418) und dem zweiten Anbringungsarm (319, 419) befindet.

3. Medizinisches Implantat (210, 310, 410) nach einem der Ansprüche 1 - 2, wobei der Humeruskopfanbringungsbereich (216, 316, 416), der Glenoidanbringungsbereich (218, 318, 418) und der Rotatorenmanschettensehnenanbringungsbereich (212, 312, 412) zusammen eine monolithische Struktur definieren.

4. Implantat (410) nach einem der Ansprüche 1 - 3, wobei der Humeruskopfanbringungsbereich (416) ein Verstärkungsglied (423) aufweist.

5. Implantat (410) nach Anspruch 4, ferner umfassend eine oder mehrere Anbringungsstützen (452), die sich von dem Verstärkungsglied (423) erstrecken, wobei die eine oder die mehreren Anbringungsstützen (452) zur Verankerung an dem Humeruskopf ausgestaltet sind.

6. Medizinisches Implantat (210, 310, 410) nach einem der vorhergehenden Ansprüche, wobei der Glenoidanbringungsbereich (218, 318, 418), der Rotatorenmanschettensehnenanbringungsbereich (212, 312, 412) und der Humeruskopfanbringungsbereich (216, 316, 416) jeweils eine oder mehrere Öffnungen (220, 222, 224, 320, 322, 324, 420, 422, 424) aufweisen, die so ausgestaltet sind, dass sich ein Anker dort hindurcherstrecken und an einem Zielort angebracht werden kann.

## Revendications

1. Implant médical (210, 310, 410) pour le traitement d'une articulation d'épaule, comprenant :
une région de fixation de tête humérale (216, 316, 416) configurée pour être fixée à la tête humérale de l'articulation d'épaule ;
une région de fixation glénoïde (218, 318, 418) configurée pour être fixée à la glène de l'articulation d'épaule ; et
une région de fixation de tendon de la coiffe des rotateurs (212, 312, 412) configurée pour être fixée au tendon de la coiffe des rotateurs de l'articulation d'épaule,
l'implant médical (210, 310, 410) étant un élément continu, la région de fixation de tête humérale (216, 316, 416) étant positionnée entre la région de fixation glénoïde (218, 318, 418) et la région de fixation de tendon de la coiffe des rotateurs (212, 312, 412) ;
l'implant médical étant configuré pour se replier sur lui-même à l'intérieur du corps à une position le long de la région de fixation de tête humérale (216, 316, 416) avec la région de fixation glénoïde (218, 318, 418) et la région de fixation de tendon de la coiffe des rotateurs (212, 312, 412) s'étendant vers le plan sagittal à partir de la région de fixation de tête humérale (216, 316, 416).

2. Implant médical (310, 410) selon la revendication 1, la région de fixation glénoïde (318, 418) étant bifurquée en un premier bras d'attache (319, 419) et un second bras d'attache (321, 421), avec un espace situé entre le premier bras d'attache (318, 418) et le second bras d'attache (319, 419).

3. Implant médical (210, 310, 410) selon l'une quelconque des revendications 1 et 2, la région de fixation de tête humérale (216, 316, 416), la région de fixation glénoïde (218, 318, 418) et la région de fixation de tendon de la coiffe des rotateurs (212, 312, 412) définissant ensemble une structure monolithique.

4. Implant (410) selon l'une quelconque des revendications 1 à 3, la région de fixation de tête humérale (416) comprenant un élément de renforcement (423).

5. Implant (410) selon la revendication 4, comprenant en outre un ou plusieurs supports de fixation (452) s'étendant à partir de l'élément de renforcement (423), le ou les supports de fixation (452) étant configurés pour être ancrés à la tête humérale.

6. Implant médical (210, 310, 410) selon n'importe quelle revendication précédente, chacune de la région de fixation glénoïde (218, 318, 418), de la région de fixation de la coiffe des rotateurs (212, 312, 412) et de la région de fixation de tête humérale (216, 316, 416) comprenant une ou plusieurs ouvertures (220, 222, 224, 320, 322, 324, 420, 422, 424) configurées pour permettre à une ancre de s'y étendre et de se fixer à un site cible.
